# EUROPEAN PATENT APPLICATION

(11) **EP 4 365 286 A1**
(43) Date of publication of application: **08.05.2024**
(21) Application number: 21951543.4
(22) Date of filing: 18.09.2021
(51) Int. Cl.: C12N 9/16, C12N 15/55, C12N 15/70, C12N 1/21, C12P 7/40, C12P 7/02, C12R 1/19

(54) **ESTERASE MUTANT AND USE THEREOF**

(30) Priority: 28.07.2021 CN 202110853890
(71) Applicant: Asymchem Laboratories (Tianjin) Co., Ltd., Tianjin 300457 (CN)
(72) Inventor: HONG, Hao, Morrisville, North Carolina 27560 (US); JAMES, Gage, Morrisville, North Carolina 27560 (US); XIAO, Yi, Tianjin 300457 (CN); ZHANG, Na, Tianjin 300457 (CN); JIAO, Xuecheng, Tianjin 300457 (CN); YANG, Yiming, Tianjin 300457 (CN); LI, Juan, Tianjin 300457 (CN); HUANG, Qixing, Tianjin 300457 (CN)
(74) Representative: HGF
(86) International application number: PCT/CN2021/119423
(87) International publication number: WO 2023/004969

(57) **Abstract**

Provided are an esterase mutant and the use thereof. The esterase mutant obtained by means of rational design and several rounds of evolution screening with enzymes on the basis of an amino acid sequence as shown in SEQ ID NO: 1 is changed in terms of protein structure and function compared with a wild-type esterase; in practical use, the catalytic activity and/or stereoselectivity of the esterase mutant is greatly improved; and when a system contains some organic cosolvents, the esterase mutant still has relatively stable catalytic activity and/or stereoselectivity. In addition, the improvement of the catalytic activity and/or stereoselectivity of the esterase mutant reduces the use amount of the enzyme to a certain extent and reduces the difficulty of post-treatment, and therefore the esterase mutant is suitable for industrial production.

## Description

### Technical Field

The present invention relates to the field of industrial enzyme preparation, in particular to an esterase mutant and a use thereof.

### Background

In the development process of pharmaceuticals, pesticides, and fine chemical industries, organic synthesis faces the larger and larger challenges. In drug molecules, often only one stereoisomer has a therapeutic effect, while the other stereoisomers do not have the therapeutic effect or even have side effects. In traditional organic synthesis, protection and deprotection steps are usually introduced to compensate for the lack of chemical and regional selectivity in reactions. However, biological enzyme catalyzed reactions typically have relatively high catalytic activity and/or stereoselectivity, and are usually performed under neutral and room temperature conditions, causing less environmental pollution. Therefore, applications of biological enzyme catalyzed technologies in the organic synthesis have a significant practical application value.

An esterase is a hydrolytic enzyme, which is a general term for enzymes that catalyze ester hydrolysis and widely present in plants, animals, and microorganisms. Animal pancreatic esterases and microbial esterases are the main sources, mainly fungi, followed by bacteria. The esterases from the different sources have different catalytic characteristics and catalytic activities. The esterase is already commercialized and plays an important role in fields such as food, medicine, and chemical engineering.

CN107058362A discloses an esterase gene est816 and a recombinant esterase thereof. It has the highly soluble expression in Escherichia coli expression system and Pichia pastoris expression system, and the recombinant esterase has a strong degradation effect on pyrethroid pesticides (including cyhalothrin, cypermethrin, fenvalerate, and deltamethrin), and the degradation rate reaches up to more than 90%. It has a broad application prospect in pyrethroid pesticide residues. CN106929493A discloses a lactonase, and its amino acid sequence is to mutate the 167-th amino acid of sequence numbered as 5 from valine to histidine, thereby the degradation efficiency of α-zearalenol is improved.

When biocatalysts are used in the organic synthesis, it is a non-natural substrate under most circumstances. However, for the non-natural substrate, its reaction activity, stability, and selectivity is often not very good, so there are not many esterases that may truly be used widely.

### Summary

A main purpose of the present invention is to provide an esterase mutant and a use thereof, as to solve problems of low esterase activity and/or low stereoselectivity in prior art.

In order to achieve the above purpose, according to one aspect of the present invention, an esterase mutant is provided, and the esterase mutant has the following mutated amino acid sequence of SEQ ID NO: 1: G19S, G19S+H86S, G19S+H86A, G19S+H86Q, G19S+H86M, G19S+H86T, G19S+H86C, G19S+H86N, G19S+F88N, G19S+F88R, G19S+F88Y, G19S+F88K, G19S+S111T, G19S+S111V, G19S+M113I, G19S+M113L, G19S+M113V, G19S+F125Y, G19S+F125S, G19S+Y128F, G19S+L157V, G19S+M166L, G19S+L187V, G19S+L187I, G19S+S218Y, G19S+S218H, G19S+S218F, G19S+S218N, G19S+H86S+S111T, G19S+H86S+S111V, G19S+H86S+M113A, G19S+H86S+M113G, G19S+H86S+M113I, G19S+H86S+M113L, G19S+H86S+M113V, G19S+H86S+M113I+L157A, G19S+H86S+M113I+L157G, G19S+H86S+M113I+L157V, G19S+H86A+S111T, G19S+H86A+S111V, G19S+H86A+M113A, G19S+H86A+M113G, G19S+H86A+M113I, G19S+H86A+M113L, G19S+H86A+M113V, G19S+H86A+M113I+S218E, G19S+H86A+M113I+S218F, G19S+H86A+M113I+S218I, G19S+H86A+M113I+S218L, G19S+H86A+M113I+S218M, G19S+H86A+M113I+S218T, G19S+H86A+M113I+S218V, G19S+H86A+M113I+S218Y, G19S+H86A+M113I+S218F+A86C, G19S+H86A+M113I+S218F+A86M, G19S+H86A+M113I+S218F+A86N, G19S+H86A+M113I+S218F+A86Q, G19S+H86A+M113I+S218F+A86S, G19S+H86A+M113I+S218F+M137F, G19S+H86A+M113I+S218F+M137L, G19S+H86A+M113I+S218F+M137Y, G19S+H86A+M113I+S218F+M137E, G19S+H86A+M113I+S218F+M137W, G19S+H86A+M113I+S218F+F219Y, G19S+H86A+M113I+S218F+F219L, G19S+H86A+M113I+S218F+F219T, G19S+H86A+M113I+S218F+F219Q, G19S+H86A+M113I+S218F+F219Y+M137F, G19S+H86A+M113I+S218F+F219Y+M137L, G19S+H86A+M113I+S218F+F219Y+M137W, G19S+H86A+M113I+S218F+F219Y+M137Y, or G19S+H86A+M113I+S218F+F219Y+M137S; or has more than 80% of identity to the mutated amino acid sequence.

Further, the amino acid sequence of the esterase mutant has more than 90%, preferably more than 95%, and more preferably more than 99% of the identity to the mutated amino acid sequence.

Further, the esterase mutant is derived from *Rauvolfia serpentina.*

According to a second aspect of the present application, a DNA molecule is provided, and the above esterase mutant is encoded.

According to a third aspect of the present application, a recombinant plasmid is provided, and the recombinant plasmid is linked to the above DNA molecule.

Further, the recombinant plasmid is selected from any one of the following: pET-21b(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, and pUC-19.

According to the fourth aspect of the present application, a non-plant host cell is provided, and the host cell contains any one of the aforementioned recombinant plasmids.

Further, the host cell is a prokaryotic cell or a eukaryotic cell, and the eukaryotic cell is a yeast cell.

Further, the host cell is a competent cell.

Further, the competent cell is an *Escherichia coli* BL21 cell or an *Escherichia coli* W3110 cell.

According to the second aspect of the present application, a method for preparing a chiral compound is provided, and the method includes: catalyzing an ester compound as shown in Formula I with the esterase mutant above mentioned to be hydrolyzed into an acid compound as shown in Formula II and an alcohol compound as shown in Formula III,.

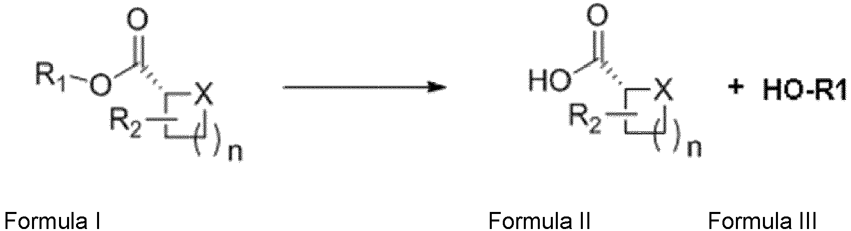

Herein, n=1, 2, 3, or 4;
X=C, O, or S;
R₁=CH₃, CH₂CH₃, CH₂-CH₂CH₃, or CHCH₃CH₃; and
R₂=H, F, Cl, Br, CH₃, or CH₂CH₃.

Further, the ester compound is any one of the following:

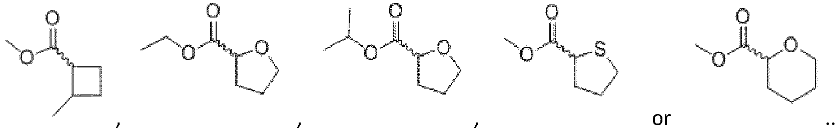

Further, the esterase mutant catalyzes the ester compound as shown in Formula I to be hydrolyzed at a temperature of 20°C to 40°C.

Further, the ester compound and the esterase are dissolved in a potassium phosphate buffer solution to form a catalytic reaction system, wherein the potassium phosphate buffer solution has a concentration of 0.1M to 1 M, and a pH value of 6.0 to 7.5.

Further, a mass ratio of the esterase mutant to the ester compound is 0.2 mg-2 mg: 20 mg.

Further, a mass ratio of the esterase mutant to the ester compound is 0.1 g~0.5 g: 10 g.

Further, the catalytic reaction system also contains a co-solvent, and the co-solvent is selected from any one of the following dimethylsulfoxide (DMSO), dichloromethane (DCM), and 2-methyltetrahydrofuran (2-MeTHF).

Further, a volume percentage content of the co-solvent in the reaction system is ≤20%.

By applying a technical scheme of the present invention, the esterase mutant obtained by means of rational design and a plurality of rounds of evolution screening with enzymes on the basis of the amino acid sequence as shown in SEQ ID NO: 1 is changed in terms of protein structure and function compared with a wild-type esterase; in practical use, the catalytic activity and/or stereoselectivity of the esterase mutant is greatly improved; and when a system contains some organic co-solvents, the esterase mutant still has relatively stable catalytic activity and/or stereoselectivity. In addition, the improvement of the catalytic activity and/or stereoselectivity of the esterase mutant reduces the usage amount of the enzyme to a certain extent and reduces the difficulty of post-treatment, and therefore the esterase mutant is suitable for industrial production.

### Detailed Description of the Embodiments

It should be noted that embodiments in the present application and features in the embodiments may be combined with each other in the case without conflicting. The present invention is described in detail below in combination with the embodiments.

In a typical embodiment, an esterase mutant is provided, and the amino acid sequence of the esterase mutant is obtained from a mutated amino acid sequence of SEQ ID NO: 1 having the following mutations: G19S, G19S+H86S, G19S+H86A, G19S+H86Q, G19S+H86M, G19S+H86T, G19S+H86C, G19S+H86N, G19S+F88N, G19S+F88R, G19S+F88Y, G19S+F88K, G19S+S111T, G19S+S111V, G19S+M113I, G19S+M113L, G19S+M113V, G19S+F125Y, G19S+F125S, G19S+Y128F, G19S+L157V, G19S+M166L, G19S+L187V, G19S+L187I, G19S+S218Y, G19S+S218H, G19S+S218F, G19S+S218N, G19S+H86S+S111T, G19S+H86S+S111V, G19S+H86S+M113A, G19S+H86S+M113G, G19S+H86S+M113I, G19S+H86S+M113L, G19S+H86S+M113V, G19S+H86S+M113I+L157A, G19S+H86S+M113I+L157G, G19S+H86S+M113I+L157V, G19S+H86A+S111T, G19S+H86A+S111V, G19S+H86A+M113A, G19S+H86A+M113G, G19S+H86A+M113I, G19S+H86A+M113L, G19S+H86A+M113V, G19S+H86A+M113I+S218E, G19S+H86A+M113I+S218F, G19S+H86A+M113I+S218I, G19S+H86A+M113I+S218L, G19S+H86A+M113I+S218M, G19S+H86A+M113I+S218T, G19S+H86A+M113I+S218V, G19S+H86A+M113I+S218Y, G19S+H86A+M113I+S218F+A86C, G19S+H86A+M113I+S218F+A86M, G19S+H86A+M113I+S218F+A86N, G19S+H86A+M113I+S218F+A86Q, G19S+H86A+M113I+S218F+A86S, G19S+H86A+M113I+S218F+M137F, G19S+H86A+M113I+S218F+M137L, G19S+H86A+M113I+S218F+M137Y, G19S+H86A+M113I+S218F+M137E, G19S+H86A+M113I+S218F+M137W, G19S+H86A+M113I+S218F+F219Y, G19S+H86A+M113I+S218F+F219L, G19S+H86A+M113I+S218F+F219T, G19S+H86A+M113I+S218F+F219Q, G19S+H86A+M113I+S218F+F219Y+M137F, G19S+H86A+M113I+S218F+F219Y+M137L, G19S+H86A+M113I+S218F+F219Y+M137W, G19S+H86A+M113I+S218F+F219Y+M137Y or G19S+H86A+M113I+S218F+F219Y+M137S; or the amino acid sequence of the esterase mutant has the mutation site in the mutated amino acid sequence, and has more than 80% (preferably more than 90%, or more than 95%, or more than 99%) of identity to the mutated amino acid sequence.

The amino acid sequence of the above esterase mutant is obtained by mutating the amino acid sequence shown in SEQ ID NO: 1, and the important site involved in the mutation includes but not limited to one or more of the following sites: G19S, H86S, H86A, H86Q, H86M, H86T, H86C, H86N, F88N, F88R, F88Y, F88K, S111T, S111V, M113A, M113G, M113I, M113L, M113V, F125Y, F125S, Y128F, M137F, M137L, M137Y, M137E, M137W, M137S, L157V, L157A, L157G, M166L, L187V, L187I, S218Y, S218H, S218F, S218N, S218E, S218I, S218L, S218M, S218T, S218V, F219Y, F219L, F219T and F219Q.

The above esterase mutant obtained by means of rational design and several rounds of evolution screening with enzymes on the basis of the amino acid sequence as shown in SEQ ID NO: 1 is changed in terms of protein structure and function compared with a wild-type esterase. In practical use, the catalytic activity and/or stereoselectivity of the esterase mutant is greatly improved; and when a system contains some organic co-solvents, the esterase mutant still has relatively stable catalytic activity and/or stereoselectivity. In addition, the improvement of the catalytic activity and/or stereoselectivity of the esterase mutant reduces the usage amount of the enzyme to a certain extent and reduces the difficulty of post-treatment, and therefore the esterase mutant is suitable for industrial production.

The amino acid sequence of SEQ ID NO: 1 (derived from *Rauvolfia serpentina*) is as follows:

MHSAANAKQQKHFVLVHGGCLGAWIWYKLKPLLESAGHKVTAVDLSAAGINPRRLDEIHTFR
DYSEPLMEVMASIPPDEKVVLLGHSFGGMSLGLAMETYPEKISVAVFMSAMMPDPNHSLTYPFEK
YNEKCPADMMLDSQFSTYGNPENPGMSMILGPQFMALKMFQNCSVEDLELAKMLTRPGSLFFQD
LAKAKKFSTERYGSVKRAYIFCNEDKSFPVEFQKWFVESVGADKVKEIKEADHMGMLSQPREVCK
CLLDISDS.

The present application optimizes a codon for Escherichia coli according to the protein sequence shown in SEQ ID NO: 1, and a specific nucleic acid sequence (namely CDS, including a termination codon taa) is SEQ ID NO: 2:

atgcacagcgctgcaaacgcaaaacaacagaagcacttcgtcctggtccacggtggttgtctgggtgcttggatctggtacaaact
gaaacctctgctggagtctgcaggtcataaagtgactgcagttgatctgagcgcagctggtatcaacccacgtcgtctggatgaaattcaca
ctttccgtgattacagcgagccactgatggaagtgatggctagcatcccgccggatgaaaaagtggttctgctgggtcattctttcggtggtat
gtctctgggtctggctatggaaacctacccggagaaaatctctgttgctgtgttcatgtccgccatgatgccggatccgaaccactctctgacc
tatccgtttgaaaagtacaacgagaagtgcccggccgatatgatgctggactctcaattctctacgtacggcaacccggaaaatccgggc
atgtctatgatcctgggcccgcagtttatggcgctgaaaatgtttcagaactgtagcgtagaagacctggaactggccaaaatgctgaccc
gtcctggctccctgtttttccaggacctggcgaaagcgaaaaagttcagcaccgaacgttatggctccgttaaacgcgcgtatattttctgca
acgaagacaaaagcttcccggttgaattccagaaatggttcgtagagtccgttggcgcggacaaagtaaaagaaatcaaagaagcgg
accacatgggcatgctgtcccagccgcgcgaagtttgcaaatgcctgctggacatttccgactcctaa.

Specific methods or steps for the rational design and enzyme evolution screening mentioned above include but are not limited to the following examples.

Firstly, a mutation site is introduced on SEQ ID NO: 1 by a mode of a full-plasmid polymerase chain reaction (PCR), to detect the activity and selectivity of the mutant, and select the mutant with the improved activity and selectivity.

The sequence SEQ ID NO: 1 is used as a template, to design a site-specific mutation primer, and a site-specific mutation means is used, to obtain a mutant plasmid with a target gene by using pET-22b (+) as an expression vector.

Herein, site-specific mutation: refers to introduction of a change (usually the change representing a favorable direction) required into a target DNA fragment (it may be a genome or a plasmid) by methods such as a polymerase chain reaction (PCR), including addition, deletion, and point mutation of bases. The site-specific mutation may quickly and efficiently improve the characters and representation of the target protein expressed by DNA, and is a very useful tool in gene research work.

The method of introducing the site-specific mutation using the full-plasmid PCR is simple and effective, and is a means currently used widely. Its principle is that: a pair of primers (forward and reverse) containing mutation sites and a template plasmid are annealed, and "cyclically extended" with a polymerase. The so-called cyclic extension refers to a cycle that the polymerase extends the primer according to the template, terminates at a 5'-end of the primer after one circle, and then undergoes repeated heating and annealing extension. This reaction is different from rolling circle amplification, and does not form a plurality of tandem copies. Extension products of the forward and reverse primers are annealed, and paired to form an open-loop plasmid with a notch. An extension product of Dpn I enzyme digestion is shredded because the original template plasmid is derived from conventional Escherichia coli, modified by dam methylation and sensitive to Dpn I. However, the plasmid with the mutated sequence synthesized in vitro is not cleaved due to the lack of methylation, so it is successfully transformed in subsequent transformations, to obtain a clone of the mutated plasmid.

On the basis of acquiring the mutant with the improved characters by a single site mutation, beneficial amino acid sites may be combined, to obtain the mutant with the better characters.

After the esterase mutant with significantly improved activity and enantioselectivity is obtained, a mode of a saturation mutation is further adopted to obtain more mutants with the better performance.

The saturation mutation is a method of acquiring a mutant with a target amino acid substituted by 19 other amino acids in a short period of time by modifying a coding gene of the target protein. This method is not only a powerful tool for protein directed modification, but also an important means for researching a protein structure-function relationship. The saturation mutation may often acquire a more ideal evolutionary body than the single site mutation. For these problems that may not be solved by the fixed site mutation method, it is precisely the unique advantage of the saturation mutation method.

The above mutant plasmid is transformed into an Escherichia coli cell and overexpressed in Escherichia coli. Then, a crude enzyme is obtained by a method of cell ultrasonication. The optimal conditions for esterase induced expression are: 25°C, and 0.06 mM IPTG induction overnight.

The mutant selected in the present application undergoes extensive experimental verification, and in the case that a substrate existing in the form of a racemate is not excessively converted, it is ultimately proved that the catalytic activity and/or stereoselectivity of the enzyme catalyzed reaction is significantly improved (such as enantiomeric excess (e.e.) increased from <10% initially to at least 80% or more), and the needs of industrial production are satisfied to a great extent.

In a typical implementation mode of the present invention, a DNA molecule is further provided, and the DNA molecule encodes any one of the above esterase mutants. The esterase mutant encoded above has the advantages of high catalytic activity and/or high stereoselectivity.

In a typical implementation mode of the present invention, a recombinant plasmid is further provided, and the recombinant plasmid is linked to the above DNA molecule. The DNA molecule may encode any one of the above esterase mutants with the high catalytic activity and/or high stereoselectivity.

In the above recombinant plasmids, any recombinant plasmids that may be used to express the DNA molecule of the above esterase is applicable to the present invention. In a preferred embodiment of the present invention, the recombinant plasmid is selected from one of the following: pET-22b (+), pET-21b (+), pET-3a (+), pET-3d (+), pET-11a (+), pET-12a (+), pET-14b, pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C. pGEX-5X-1, pGEX-6p-1, pGEX-6p-2, pBV220, pBV221, pBV222, pTrc99A, pTwin1, pEZZ18, pKK232-8, pUC-18 and pUC-19.

In a typical implementation mode of the present invention, a non-plant host cell is further provided, and the host cell contains any one of the above recombinant plasmids. The specific host cell may be a prokaryotic cell or a eukaryotic cell, and preferably the eukaryotic cell is a yeast cell. More preferably, the above host cell is a competent cell, and further preferably, the competent cell is an *Escherichia coli* BL21 cell or an *Escherichia coli* W3110 cell.

In a typical implementation mode of the present invention, a preparation method for a chiral compound is further provided, and the preparation method includes: the aforementioned esterase mutant is used to catalyze an ester compound shown in Formula I to hydrolyze into an acid compound shown in Formula II and an alcohol compound shown in Formula III.

In a preferred embodiment, the ester compound is any one of the following:

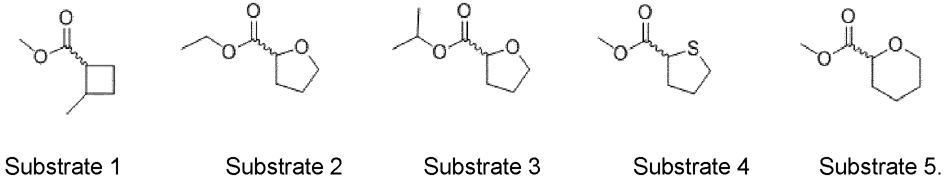

In a preferred embodiment, the esterase mutant has the relatively high catalytic activity or stereoselectivity at a temperature of 20°C~40°C, thus it may catalyze a hydrolysis reaction of the ester compound shown in Formula I.

In a preferred embodiment, a potassium phosphate buffer, the ester compound, and the esterase form a catalytic reaction system, herein the concentration of the potassium phosphate buffer is preferably 0.1 M~1 M, and the pH value is preferably 6.0-7.5. Under this condition, it may catalyze different substrates and has the relatively high activity and/or stereoselectivity.

In a preferred embodiment, the mass ratio of the esterase mutant to the ester compound is 0.2 mg-2 mg: 20 mg, it is suitable for a small reaction volume (such as 0.6 mL) in this mass ratio, and when the corresponding substrate amount is 20 mg, the mass range of the enzyme is 2 mg~0.2 mg.

In another preferred embodiment, the mass ratio of the esterase mutant to the ester compound is 0.1 g~0.5 g: 10 g. This usage ratio is particularly suitable for a large reaction system (such as 100 mL), and when the corresponding substrate amount is 10 g, the mass range of the enzyme is 0.1 g~0.5 g.

The mutant obtained by evolutionary screening in the present application may not only catalyze the substrate hydrolysis reaction under suitable buffer conditions, but also has the relatively high stability, catalytic activity, and/or stereoselectivity in the presence of certain co-solvents. In a preferred embodiment, the reaction system also contains a co-solvent, and the co-solvent is selected from any one of the following: DMSO, DCM, and 2-MeTHF. In the reaction systems in which these co-solvents exist, the catalytic activity and/or stereoselectivity of the mutant in the present application are still relatively stable.

In a preferred embodiment, the volume percentage content of the co-solvent in the reaction system is ≤20%, and the mutant of the present application still has the relatively high catalytic activity and/or stereoselectivity when the reaction system contains this content of the co-solvent.

The beneficial effects of the present application are further described below in combination with specific embodiments. It should be noted that the substrates used in the following embodiments are shown in Substrates 1 to 5 above.

### Embodiment 1

20 mg of Substrate 1 was added into a reaction system of 0.6 mL, including 2 mg of esterase, and 0.3 M of potassium phosphate buffer with pH 7.5. After it was reacted at 20°C for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 2 and Substrate 3, as described in Substrate 1, established reaction and treatment modes of the same system. Results were shown in Table 1.

**Table 1**

| Enzyme | Substrate 1 | | Substrate 2 | | Substrate 3 | |
|---|---|---|---|---|---|---|
| | Activity | e.e.(%) | Activity | e.e.(%) | Activity | e.e.(%) |
| WT | 20 | 30 | 10 | 35 | 15 | 33 |
| G19S | ++ | ** | + | *** | ++ | *** |
| G19S+H86S | - | ** | ++ | *** | + | ** |
| G19S+H86A | ++ | *** | + | **** | - | *** |
| G19S+H86Q | - | ** | - | *** | ++ | ** |
| G19S+H86M | ++ | ** | + | *** | + | ** |
| G19S+H86T | - | ** | - | *** | ++ | *** |
| G19S+H86C | + | ** | ++ | *** | - | ** |
| G19S+H86N | ++ | *** | - | ** | - | ** |
| G19S+F88N | + | * | ++ | *** | + | *** |
| G19S+F88R | + | *** | - | # | ++ | ** |
| G19S+F88Y | ++ | * | - | *** | + | # |
| G19S+F88K | - | *** | ++ | ** | - | ** |
| G19S+S111T | ++ | ** | - | *** | + | # |
| G19S+S111V | + | ** | - | *** | ++ | ** |
| G19S+M113I | +++ | *** | + | *** | - | *** |
| G19S+M113L | + | *** | - | *** | ++ | ** |
| G19S+M113V | - | ** | ++ | ** | + | *** |
| G19S+F125Y | ++ | # | + | ** | + | *** |
| G19S+F125S | - | ** | ++ | *** | - | *** |
| G19S+Y128F | - | *** | - | ** | ++ | ** |
| G19S+L157V | ++ | *** | - | * | - | ** |
| G19S+M166L | + | ** | - | # | ++ | *** |
| G19S+L187V | ++ | ** | - | *** | + | *** |
| G19S+L187I | - | *** | ++ | ** | + | ** |
| G19S+S218Y | + | ** | ++ | *** | - | ** |
| G19S+S218H | ++ | ** | + | *** | - | # |
| G19S+S218F | + | *** | +++ | **** | + | *** |
| G19S+S218N | - | ** | - | *** | ++ | ** |

The meanings of the catalytic activity values of the wild-type esterase on different substrates were the same in each embodiment, namely, the activity represented the transformation rate. Here, the activity value of Substrate 1, which was 20, was used as an example to describe: under the conditions of 0.3 M potassium phosphate buffer with pH 7.0, 20°C, 2 mg of the wild-type enzyme, for 3 h, the transformation rate of Substrate 1 was 20%.

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

The ee value less than 0% (it referred to the ee value of the product, since the substrate was racemic, namely both S configuration and R configuration, for example each accounted for about 50%. In the catalytic process, the enzyme might selectively catalyze S and R configurations, such as the transformation of more substrates with the R configurations were selectively catalyzed, there were more corresponding R configurations in the product. If, during evolution, a certain mutated enzyme catalyzed the R configuration, an unexpected configuration might appear, namely the proportion (such as 40%) of the product with the R configuration was lower than the proportion of the product with the S configuration (such as 60%), then the ee value (namely -20%) of the product with the R configuration was less than 0) was marked as #, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

### Embodiment 2

20 mg of Substrate 4 was added into a reaction system of 0.6 mL, including 2 mg of esterase, and 0.3 M of potassium phosphate buffer with pH 7.0. After it was reacted at 20°C for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 5, as described in Substrate 1, established reaction and treatment modes of the same system. Results were shown in Table 2.

**Table 2**

| Enzyme | Substrate 4 | | Substrate 5 | |
|---|---|---|---|---|
| | Activity | e.e.(%) | Activity | e.e.(%) |
| WT | 20 | 10 | 25 | 20 |
| G19S | ++ | ** | + | *** |
| G19S+H86S | ++ | ** | - | *** |
| G19S+H86A | + | ** | ++ | *** |
| G19S+H86Q | ++ | ** | - | *** |
| G19S+H86M | + | ** | ++ | *** |
| G19S+H86T | - | ** | ++ | *** |
| G19S+H86C | ++ | ** | - | *** |
| G19S+H86N | + | *** | ++ | ** |
| G19S+F88N | + | *** | ++ | # |
| G19S+F88R | ++ | ** | - | *** |
| G19S+F88Y | - | # | ++ | *** |
| G19S+F88K | + | *** | ++ | ** |
| G19S+S111T | ++ | *** | - | ** |
| G19S+S111V | ++ | ** | - | *** |
| G19S+M113I | + | *** | ++ | *** |
| G19S+M113L | ++ | ** | - | *** |
| G19S+M113V | - | *** | ++ | ** |
| G19S+F125Y | ++ | *** | - | ** |
| G19S+F125S | - | ** | ++ | *** |
| G19S+Y128F | ++ | *** | - | ** |
| G19S+L157V | ++ | *** | - | # |
| G19S+M166L | + | ** | ++ | *** |
| G19S+L187V | - | *** | ++ | ** |
| G19S+L187I | ++ | *** | - | ** |
| G19S+S218Y | ++ | ** | + | *** |
| G19S+S218H | - | ** | ++ | *** |
| G19S+S218F | ++ | *** | + | *** |
| G19S+S218N | ++ | ** | - | *** |

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5 times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

The mutation was continued on the basis of the above results, as to improve the ee value of the product.

### Embodiment 3

20 mg of Substrate 1 was added into a reaction system of 0.6 mL, including 2 mg of esterase, and 0.3 M of potassium phosphate buffer with pH 7.0. After it was reacted at 20°C for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 2 and Substrate 3, as described in Substrate 1, established reaction and treatment modes of the same system. Results were shown in Table 3.

**Table 3**

| Enzyme | Substrate 1 | | Substrate 2 | | Substrate 3 | |
|---|---|---|---|---|---|---|
| | Activity | e.e.(%) | Activity | e.e.(%) | Activity | e.e.(%) |
| G19S+H86S+S111T | - | *** | +++ | **** | - | *** |
| G19S+H86S+S111V | +++ | *** | - | *** | + | **** |
| G19S+H86S+M113A | ++ | **** | ++ | *** | + | *** |
| G19S+H86S+M113G | - | *** | + | **** | ++ | *** |
| G19S+H86S+M113I | + | **** | +++ | **** | ++ | **** |
| G19S+H86S+M113L | +++ | *** | + | **** | - | *** |
| G19S+H86S+M113V | + | *** | ++ | **** | ++ | *** |
| G19S+H86S+M113I+L157A | - | *** | ++ | *** | - | **** |
| G19S+H86S+M113I+L157G | ++ | **** | - | *** | - | *** |
| G19S+H86S+M113I+L157V | - | *** | + | **** | ++ | *** |
| G19S+H86A+S111T | + | *** | ++ | **** | ++ | *** |
| G19S+H86A+S111V | +++ | *** | + | ** | + | **** |
| G19S+H86A+M113A | + | *** | ++ | **** | + | *** |
| G19S+H86A+M113G | ++ | *** | + | **** | + | *** |
| G19S+H86A+M113I | + | *** | +++ | *** | ++ | **** |
| G19S+H86A+M113L | +++ | **** | + | *** | ++ | *** |
| G19S+H86A+M113V | + | *** | ++ | **** | + | *** |
| G19S+H86A+M113I+S218E | + | *** | + | **** | ++ | *** |
| G19S+H86A+M113I+S218F | ++ | **** | +++ | *** | ++ | **** |
| G19S+H86A+M113I+S218I | ++ | *** | + | **** | ++ | *** |
| G19S+H86A+M113I+S218L | - | *** | - | *** | ++ | **** |
| G19S+H86A+M113I+S218M | ++ | *** | ++ | **** | + | *** |
| G19S+H86A+M113I+S218T | + | **** | ++ | *** | ++ | *** |
| G19S+H86A+M113I+S218V | ++ | *** | + | *** | ++ | **** |
| G19S+H86A+M113I+S218Y | ++ | *** | + | **** | + | *** |

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

### Embodiment 4

20 mg of Substrate 4 was added into a reaction system of 0.6 mL, including 2 mg of esterase, and 0.3 M of potassium phosphate buffer with pH 7.0. After it was reacted at 20°C for 3 h, 1.5 mL of anhydro into a reaction system of 0.6 mL, including us ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 5, as described in Substrate 1, established reaction and treatment modes of the same system. Results were shown in Table 4.

**Table 4**

| Enzyme | Substrate 4 | | Substrate 5 | |
|---|---|---|---|---|
| | Activity | e.e.(%) | Activity | e.e.(%) |
| G19S+H86S+S111T | - | *** | ++ | **** |
| G19S+H86S+S111V | ++ | **** | - | *** |
| G19S+H86S+M113A | ++ | *** | + | **** |
| G19S+H86S+M113G | - | **** | ++ | *** |
| G19S+H86S+M113I | + | **** | +++ | **** |
| G19S+H86S+M113L | +++ | **** | + | **** |
| G19S+H86S+M113V | + | **** | ++ | **** |
| G19S+H86S+M113I+L157A | - | **** | ++ | **** |
| GI9S+H86S+M113I+L157G | ++ | **** | - | **** |
| G19S+H86S+M113I+L157V | - | **** | ++ | **** |
| G19S+H86A+S111T | ++ | **** | ++ | *** |
| G19S+H86A+S111V | ++ | **** | + | *** |
| G19S+H86A+M113A | ++ | *** | + | **** |
| G19S+H86A+M113G | + | **** | ++ | *** |
| G19S+H86A+M113I | + | **** | +++ | **** |
| G19S+H86A+M113L | ++ | **** | + | **** |
| G19S+H86A+M113V | + | **** | ++ | **** |
| G19S+H86A+M113I+S218E | ++ | **** | + | **** |
| G19S+H86A+M113I+S218F | ++ | **** | ++ | **** |
| G19S+H86A+M113I+S2181 | ++ | **** | + | **** |
| G19S+H86A+M113I+S218L | - | *** | ++ | **** |
| G19S+H86A+M113I+S218M | + | **** | ++ | *** |
| G19S+H86A+M113I+S218T | + | *** | ++ | **** |
| G19S+H86A+M113I+S218V | ++ | **** | + | *** |
| G19S+H86A+M113I+S218Y | ++ | **** | + | *** |

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

The following embodiments further combined the beneficial mutation sites, as to further improve the ee value of the product.

### Embodiment 5

20 mg of Substrate 1 was added into a reaction system of 0.6 mL, including 1 mg of esterase, and 0.3 M of potassium phosphate buffer with pH 6.5. After it was reacted at 20°C for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 2 and Substrate 3, as described in Substrate 1, established reaction and treatment modes of the same system. Results were shown in Table 5.

**Table 5**

| Enzyme | Substrate 1 | | Substrate 2 | | Substrate 3 | |
|---|---|---|---|---|---|---|
| | Activity | e.e.(%) | Activity | e.e.(%) | Activity | e.e.(%) |
| G19S+H86A+M113I+S218F+A86C | ++ | **** | ++ | **** | ++ | ****** |
| G19S+H86A+M113I+S218F+A86M | +++ | **** | +++ | ****** | ++ | **** |
| G19S+H86A+M113I+S218F+A86N | ++ | ****** | + | ***** | + | **** |
| G19S+H86A+M113I+S218F+A86Q | + | **** | ++ | **** | + | ****** |
| G19S+H86A+M113I+S218F+A86S | ++ | ****** | + | ***** | ++ | **** |
| G19S+H86A+M113I+S218F+M137F | + | **** | + | **** | ++ | ***** |
| G19S+H86A+M1131+S218F+M137L | + | ***** | ++ | ***** | + | **** |
| G19S+H86A+M113I+S218F+M137Y | +++ | **** | ++ | **** | ++ | ***** |
| G19S+H86A+M113I+S218F+M137E | ++ | ***** | ++ | ***** | ++ | **** |
| G19S+H86A+M113I+S218F+M137W | +++ | **** | ++ | ***** | +++ | ***** |
| G19S+H86A+M113I+S218F+F219Y | +++ | ****** | + | ****** | + | ****** |
| G19S+H86A+M113I+S218F+F219L | ++ | ***** | ++ | ***** | ++ | **** |
| G19S+H86A+M113I+S218F+F219T | +++ | ***** | ++ | **** | ++ | **** |
| G19S+H86A+M113I+S218F+F219Q | + | **** | ++ | ***** | + | ***** |
| G19S+H86A+M113I+S218F+F219Y+M137F | ++ | ****** | +++ | ****** | +++ | ****** |
| G19S+H86A+M113I+S218F+F219Y+M137L | +++ | ****** | ++ | ****** | +++ | ****** |
| G19S+H86A+M113I+S218F+F219Y+M137W | ++ | ****** | ++ | ****** | +++ | ****** |
| G19S+H86A+M113I+S218F+F219Y+M137Y | +++ | ****** | +++ | ****** | ++ | ****** |
| G19S+H86A+M113I+S218F+F219Y+M137S | ++ | ****** | ++ | ****** | +++ | ****** |

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

### Embodiment 6

20 mg of Substrate 4 was added into a reaction system of 0.6 mL, including 1 mg of esterase, and 0.3 M of potassium phosphate buffer with pH 6.5. After it was reacted at 20°C for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Substrate 5, as described in Substrate 1, established reaction and treatment modes of the same system. Results were shown in Table 6.

**Table 6**

| Enzyme | Substrate 4 | | Substrate 5 | |
|---|---|---|---|---|
| | Activity | e.e.(%) | Activity | e.e.(%) |
| G19S+H86A+M113I+S218F+A86C | +++ | ***** | ++ | ***** |
| G19S+H86A+M113I+S218F+A86M | ++ | **** | ++ | ***** |
| G19S+H86A+M113I+S218F+A86N | +++ | ***** | ++ | **** |
| G19S+H86A+M113I+S218F+A86Q | + | **** | ++ | **** |
| G19S+H86A+M113I+S218F+A86S | +++ | ***** | + | **** |
| G19S+H86A+M113I+S218F+M137F | ++ | **** | + | **** |
| G19S+H86A+M113I+S218F+M137L | ++ | **** | ++ | **** |
| G19S+H86A+M113I+S218F+M137Y | ++ | **** | + | **** |
| G19S+H86A+M113I+S218F+M137E | ++ | ***** | + | ***** |
| G19S+H86A+M113I+S218F+M137W | ++ | **** | +++ | ***** |
| G19S+H86A+M113I+S218F+F219Y | ++ | ***** | + | ****** |
| G19S+H86A+M113I+S218F+F219L | ++ | ***** | ++ | ***** |
| G19S+H86A+M113I+S218F+F219T | +++ | ***** | + | ***** |
| G19S+H86A+M113IS218F+F219Q | + | ***** | ++ | ***** |
| G19S+H86A+M113I+S218F+F219Y+M137F | ++ | ****** | ++ | ****** |
| G19S+H86A+M113I+S218F+F219Y+M137L | ++ | ****** | ++ | ****** |
| G19S+H86A+M113I+S218F+F219Y+M137W | ++ | ****** | +++ | ***** |
| G19S+H86A+M113I+S218F+F219Y+M137Y | +++ | ****** | +++ | ****** |
| G19S+H86A+M113I+S218F+F219Y+M137S | ++ | ****** | ++ | ****** |

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

Based on the reaction conditions in the aforementioned embodiments, the following embodiments further optimized the reaction system.

### Embodiment 7

20 mg of Substrate 1 was added into a reaction system of 0.6 mL, including 1 mg of esterase (G19S+H86A+M113I+S218F+F219Y+M137L), and 0.3 M of potassium phosphate buffer with pH 6.5. Based on the reaction conditions, a reaction system was optimized with co-solvents 2-MeTHF (0%-20%), DCM (0%-20%), and DMSO (0%-20%) with different solubility, a buffer with different concentrations (0.1 M∼1 M potassium phosphate buffer pH 6.5), a buffer with different pHs (0.3 M potassium phosphate buffer pH 6.0-7.5), and reaction temperature (20°C ~40°C). After it was reacted for 3 h, 1.5 mL of anhydrous ethanol was added into 0.6 mL of the reaction system. After being shaken fully, an appropriate amount of anhydrous magnesium sulfate was added, it was centrifuged at 12000 rpm for 3 min, a supernatant was taken and diluted twice with the anhydrous ethanol for liquid phase detection, the transformation rate and e.e. value were detected. Results were shown in Tables 7-10.

**Table 7**

| Enzyme | Co-solvent | Substrate1 | |
|---|---|---|---|
| | | Activity | e.e.(%) |
| G19S+H86A+M113I+S218F+F219Y+M137L | DMSO 0% | +++ | ****** |
| G19S+H86A+M113I+S218F+F219Y+M137L | DMSO 5% | +++ | ****** |
| G19S+H86A+M113I+S218F+F219Y+M137L | DMSO 10% | ++ | ****** |
| G19S+H86A+M113I+S218F+F219Y+M137L | DMSO 15% | ++ | ****** |
| G19S+H86A+M113I+S218F+F219Y+M137L | DMSO 20% | ++ | ****** |
| G19S+H86A+M113I+S218F+F219Y+M137L | DCM 0% | +++ | ****** |
| G19S+H86A+M113I+S218F+F219Y+M137L | DCM 5% | +++ | ****** |
| G19S+H86A+M113I+S218F+F219Y+M137L | DCM 10% | ++ | ****** |
| G19S+H86A+M113I+S218F+F219Y+M137L | DCM 15% | ++ | ****** |
| G19S+H86A+M113I+S218F+F219Y+M137L | DCM 20% | ++ | ****** |
| G19S+H86A+M113I+S218F+F219Y+M137L | 2-MeTHF 0% | +++ | ****** |
| G19S+H86A+M113I+S218F+F219Y+M137L | 2-MeTHF 5% | + | ****** |
| G19S+H86A+M113I+S218F+F219Y+M137L | 2-MeTHF 10% | + | ****** |
| G19S+H86A+M113I+S218F+F219Y+M137L | 2-MeTHF 15% | - | ****** |
| G19S+H86A+M113I+S218F+F219Y+M137L | 2-MeTHF 20% | - | ****** |

Note: the reaction conditions in Table 7 were as follows: the different co-solvents were contained, the buffer was 0.3 M potassium phosphate buffer with pH 6.5, and the reaction temperature was 20°C.

**Table 8**

| Enzyme | Buffer | Substrate 1 | |
|---|---|---|---|
| | | Activity | e.e.(%) |
| G19S+H86A+M113I+S218F+F219Y+M137L | 0.1M potassium phosphate buffer pH6.5 | ++ | ****** |
| G19S+H86A+M113I+S218F+F219Y+M137L | 0.3M potassium phosphate buffer pH6.5 | +++ | ****** |
| G19S+H86A+M113I+S218F+F219Y+M137L | 0.5M potassium phosphate buffer pH6.5 | +++ | **** |
| G19S+H86A+M113I+S218F+F219Y+M137L | 0.7M potassium phosphate buffer pH6.5 | +++ | *** |
| G19S+H86A+M113I+S218F+F219Y+M137L | 1M potassium phosphate buffer pH6.5 | +++ | *** |

Note: the reaction conditions in Table 8 were as follows: the co-solvent was not contained, and the reaction temperature was 20°C.

**Table 9**

| Enzyme | Buffer | Substrate 1 | |
|---|---|---|---|
| | | Activity | e.e.(%) |
| G19S+H86A+M113I+S218F+F219Y+M137L | 0.3M potassium phosphate buffer pH6.0 | ++ | ****** |
| G19S+H86A+M113I+S218F+F219Y+M137L | 0.3M potassium phosphate buffer pH6.5 | +++ | ****** |
| G19S+H86A+M113I+S218F+F219Y+M137L | 0.3M potassium phosphate buffer pH7.0 | +++ | ***** |
| G19S+H86A+M113I+S218F+F219Y+M137L | 0.3M potassium phosphate buffer pH7.5 | +++ | **** |

Note: the reaction conditions in Table 9 were as follows: the co-solvent was not contained, the buffer was 0.3 M potassium phosphate buffer, and the reaction temperature was 20°C.

**Table 10**

| Enzyme | Reaction temperature | Substrate1 | |
|---|---|---|---|
| | | Activity | e.e.(%) |
| G19S+H86A+M113I+S218F+F219Y+M137L | 20°C | +++ | ****** |
| G19S+H86A+M113I+S218F+F219Y+M137L | 25°C | +++ | ***** |
| G19S+H86A+M113I+S218F+F219Y+M137L | 30°C | ++++ | **** |
| G19S+H86A+M113I+S218F+F219Y+M137L | 35°C | +++ | *** |
| G19S+H86A+M113I+S218F+F219Y+M137L | 40°C | ++ | *** |

Note: the reaction conditions in Table 10 were as follows: the co-solvent was not contained, the buffer was 0.3 M potassium phosphate buffer, and pH was 6.5.

Compared with a female parent, the times of decrease and increase in activity were as follows: --- represented decrease by less than 5 times, -- represented decrease by 2-5 times, - represented decrease by 1-2 times, + represented increase by 1-2 times, ++ represented increase by 2-5times, +++ represented increase by 5-10 times, and ++++ represented increase by more than 10 times.

# represented the ee value less than 0%, * represented the ee value of 0-50%, ** represented the ee value of 50-60%, *** represented the ee value of 60-70%, **** represented the ee value of 70-80%, ***** represented the ee value of 80-95%, and ****** represented the ee value greater than 95%.

An amplification reaction was performed on 10 g of the substrate.

### Embodiment 8

Based on the optimized reaction system, the amplification reaction was performed. 10 g of Substrate 1 was added into a reaction system of 100 mL, including 100 mg of esterase (G19S+H86A+M113I+S218F+F219Y+M137L), and 0.3 M of potassium phosphate buffer with pH 6.5. It was reacted at 20°C, the reaction time was tracked for sampling and detection, and pH was adjusted to about 6.4. After 3 h of the reaction, the transformation rate was 49%, and the e.e. value was 99%. After a reaction sample was post-treated, 100 mL of dichloromethane was added for extraction. After being shaken fully, an organic layer was separated, and an appropriate amount of anhydrous sodium sulfate was added for further filtration. The organic layer was also subjected to rotary evaporation treatment. Finally, 4.8 g of the sample was obtained, the purity was 98%, and the e.e. value was 98%. Nuclear magnetic detection was further performed, and the yield was 45%.

### Embodiment 9

Based on the optimized reaction system, the amplification reaction was performed. 10 g of Substrate 4 was added into a reaction system of 100 mL, 100 mg of esterase (G19S+H86A+M113I+S218F+F219Y+M137L), and 0.3 M of potassium phosphate buffer pH 6.5. It was reacted at 20°C, the reaction time was tracked for sampling and detection, and pH was adjusted to about 6.4. After 6 h of the reaction, the transformation rate was 49%, and the e.e. value was 99%. After a reaction sample was post-treated, 100 mL of dichloromethane was added for extraction. After being shaken fully, an organic layer was separated, and an appropriate amount of anhydrous sodium sulfate was added for further filtration. The organic layer was also subjected to rotary evaporation treatment. Finally, 4.6 g of the sample was obtained, the purity was 98%, and the e.e. value was 98%. Nuclear magnetic detection was further performed, and the yield was 44%.

From the above description, it may be seen that the embodiments of the present invention achieve the following technical effects: the esterase mutant obtained by means of rational design and a plurality of rounds of evolution screening with enzymes on the basis of an amino acid sequence as shown in SEQ ID NO: 1 is changed in terms of protein structure and function compared with a wild-type esterase; in practical use, the catalytic activity and/or stereoselectivity of the esterase mutant is greatly improved; and when a system contains some organic co-solvents, the esterase mutant still has relatively stable catalytic activity and/or stereoselectivity. In addition, the improvement of the catalytic activity and/or stereoselectivity of the esterase mutant reduces the usage amount of the enzyme to a certain extent and reduces the difficulty of post-treatment, and therefore the esterase mutant is suitable for industrial production.

The above are only preferred embodiments of the present invention and are not intended to limit the present invention. For those skilled in the art, the present invention may have various modifications and variations. Any modifications, equivalent replacements, improvements and the like made within the spirit and principles of the present invention shall be included within the scope of protection of the present invention.

## Claims

1. An esterase mutant, wherein the esterase mutant comprising:
(a) a protein having the amino acid sequence of SEQ ID NO: 1 with a mutation of one or more amino acids and having a esterase activity, wherein the mutation comprises any one or more of the group consisting of: G19S, G19S+H86S, G19S+H86A, G19S+H86Q, G19S+H86M, G19S+H86T, G19S+H86C, G19S+H86N, G19S+F88N, G19S+F88R, G19S+F88Y, G19S+F88K, G19S+S111T, G19S+S111V, G19S+M113I, G19S+M113L, G19S+M113V, G19S+F125Y, G19S+F125S, G19S+Y128F, G19S+L157V, G19S+M166L, G19S+L187V, G19S+L187I, G19S+S218Y, G19S+S218H, G19S+S218F, G19S+S218N, G19S+H86S+S111T, G19S+H86S+S111V, G19S+H86S+M113A, G19S+H86S+M113G, G19S+H86S+M113I, G19S+H86S+M113L, G19S+H86S+M113V, G19S+H86S+M113I+L157A, G19S+H86S+M113I+L157G, G19S+H86S+M113I+L157V, G19S+H86A+S111T, G19S+H86A+S111V, G19S+H86A+M113A, G19S+H86A+M113G, G19S+H86A+M113I, G19S+H86A+M113L, G19S+H86A+M113V, G19S+H86A+M113I+S218E, G19S+H86A+M113I+S218F, G19S+H86A+M113I+S218I, G19S+H86A+M113I+S218L, G19S+H86A+M113I+S218M, G19S+H86A+M113I+S218T, G19S+H86A+M113I+S218V, G19S+H86A+M113I+S218Y, G19S+H86A+M113I+S218F+A86C, G19S+H86A+M113I+S218F+A86M, G19S+H86A+M113I+S218F+A86N, G19S+H86A+M113I+S218F+A86Q, G19S+H86A+M113I+S218F+A86S, G19S+H86A+M113I+S218F+M137F, G19S+H86A+M113I+S218F+M137L, G19S+H86A+M113I+S218F+M137Y, G19S+H86A+M113I+S218F+M137E, G19S+H86A+M113I+S218F+M137W, G19S+H86A+M113I+S218F+F219Y, G19S+H86A+M113I+S218F+F219L, G19S+H86A+M113I+S218F+F219T, G19S+H86A+M113I+S218F+F219Q, G19S+H86A+M113I+S218F+F219Y+M137F, G19S+H86A+M113I+S218F+F219Y+M137L, G19S+H86A+M113I+S218F+F219Y+M137W, G19S+H86A+M113I+S218F+F219Y+M137Y or G19S+H86A+M113I+S218F+F219Y+M137S; or
(b) a protein has an amino acid sequence having 80% or higher identity to the protein of (a).

2. The esterase mutant according to claim 1, wherein the esterase mutant has an amino acid sequence having 90% or higher, preferably 95% or higher, and more preferably 99% or higher identity to the protein of (a).

3. The esterase mutant according to claim 1 or 2, wherein the esterase mutant is derived from *Rauvolfia serpentina.*

4. A DNA molecule, coding the esterase mutant according to claims 1-3.

5. A recombinant plasmid, connected with the DNA molecule according to claim 4.

6. The recombinant plasmid according to claim 5, wherein the recombinant plasmid is selected from any one of the group consisting of : pET-21b(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18 and pUC-19.

7. A non-plant host cell, comprising the recombinant plasmid according to claim 5 or 6.

8. The host cell according to claim 7, wherein the host cell is a prokaryotic cell or an eukaryotic cell, and the eukaryotic cell is a yeast cell.

9. The host cell according to claim 8, wherein the host cell is a competent cell.

10. The host cell according to claim 9, wherein the competent cell is an *Escherichia coli* BL21 cell or an *Escherichia coli* W3110.

11. A method for preparing a chiral compound, comprising:
catalyzing an ester compound as shown in Formula I with the esterase mutant according to any one of claims 1 -3 to be hydrolyzed into an acid compound as shown in Formula II and an alcohol compound as shown in Formula III, 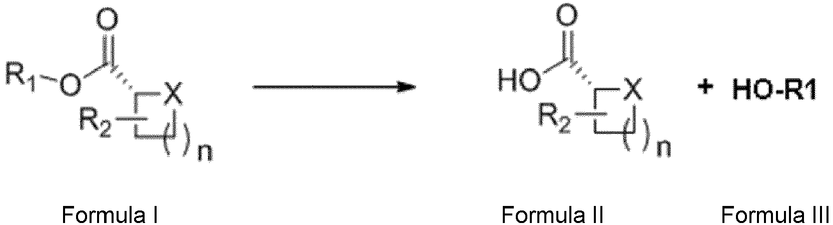
wherein n=1, 2, 3 or 4;
X=C, OorS;
R₁=CH₃, CH₂CH₃, CH₂-CH₂CH₃ or CHCH₃CH₃; and
R₂=H, F, Cl, Br, CH₃ or CH₂CH₃.

12. The method according to claim 11, wherein the ester compound is selected any one of the group consisting of:

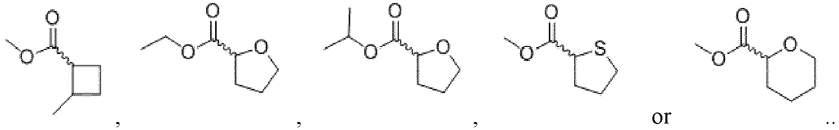

13. The method according to claim 11, wherein the esterase mutant catalyzes the ester compound as shown in Formula I to be hydrolyzed at a temperature of 20°C to 40°C.

14. The method according to claim 11, wherein the ester compound and the esterase are dissolved in a potassium phosphate buffer solution to form a catalytic reaction system, wherein the potassium phosphate buffer solution has a concentration of 0.1M to 1 M, and a pH value of 6.0 to 7.5.

15. The method according to claim 11, wherein a mass ratio of the esterase mutant to the ester compound is 0.2 mg-2 mg: 20 mg.

16. The method according to claim 11, wherein a mass ratio of the esterase mutant to the ester compound is 0.1 mg-0.5 g: 10 g.

17. The method according to claim 14, wherein the catalytic reaction system further comprises a cosolvent, and the cosolvent is selected from any one of the group consisting of DMSO, DCM and 2-MeTHF.

18. The method according to claim 17, wherein a volume percentage of the cosolvent in the reaction system is at most 20%.
